# EUROPÄISCHE PATENTSCHRIFT

(11) **EP 2 752 176 B1**
(45) Veröffentlichungstag und Bekanntmachung des Hinweises auf die Patenterteilung: **20.03.2024**
(21) Anmeldenummer: 13150513.3
(22) Anmeldetag: 08.01.2013
(51) Int. Cl.: A61F 13/02, A61M 16/04, A61M 25/02

(54) **Antimikrobiell aktive Wundauflage für Katheterfixierungen**
Antimicrobially active wound dressing for fixing catheters
Pansement actif antimicrobien pour fixations de cathéter

(43) Veröffentlichungstag der Anmeldung: 09.07.2014
(73) Patentinhaber: BSN medical GmbH, 22761 Hamburg (DE)
(72) Erfinder: Schütz, Patrick, 21075 Hamburg (DE); Schultz, Bettina, 22455 Hamburg (DE)
(74) Vertreter: Uexküll & Stolberg

(56) Entgegenhaltungen:
- US-A- 4 617 326
- US-A- 5 973 221
- US-A1- 2004 249 328
- US-A1- 2006 129 080
- US-A1- 2010 331 785
- US-A1- 2012 004 615

## Beschreibung

Die vorliegende Erfindung betrifft eine mehrschichtige Wundauflage gemäß dem Oberbegriff des beigefügten Anspruchs 1 sowie deren Verwendung zur Abdeckung von Wunden, insbesondere zur Abdeckung von Wundöffnungen bei in den Körper hinein- oder aus dem Körper herausführenden Leitungen und Schläuchen, wie z.B. Kathetern.

Wundauflagen, sog. Gateway Dressings, die dazu geeignet sind, in den Körper hinein- oder aus dem Körper herausführende Leitungen und Schläuche, wie beispielsweise Katheter, zu fixieren, die durch die Leitungen oder Schläuche verursachte Wundöffnung abzudecken und das Wundsekret aufzunehmen, sind bekannt.

Üblicherweise werden zu diesem Zweck Wundauflagen aus einem Vliesstoff oder einem Gewebe, sogenannte Gaze, als Schlitzkompressen angeboten, die einen oder mehrere Schlitze aufweisen, um die Wundauflage um Leitungen oder Schläuche aus einer menschlichen oder tierischen Körperöffnung herumgelegen zu können. Die Hauptaufgabe solcher Produkte liegt in der Abdeckung der Wundöffnung. Nachteilig bei bekannten Schlitzkompressen ist, dass sie die Wundöffnung häufig nur unzureichend verschließen und auf Grund ihrer beschränkten Aufnahmekapazität von Wundflüssigkeit öfter ausgewechselt werden müssen.

US 2006/12080 A1 beschreibt eine Wundauflage, die flächig ausgebildet ist und einen umlaufenden Rand aufweist, wobei die Wundauflage eine hydrophobe antimikrobielle Schicht, eine hydrophile absorbierende Schicht und eine Deckschicht aufweist, wobei ausgehend vom Rand der Wundauflage mindestens ein Einschnitt ausgebildet ist, der die Wundauflage teilweise trennt. Die hydrophile absorbierende Schicht kann aus Cellulose, Hygienepapier, quervernetzten Polysacchariden, Vinylpolymeren oder Acrylpolymeren gebildet sein.

US-Patent 5 973 221 offenbart eine Wundauflage, die flächig ausgebildet ist und einen umlaufenden Rand aufweist, wobei die Wundauflage eine hydrophile antiseptische Wundkontaktschicht, eine flüssigkeitsabsorbierende Schicht aus elastisch zusammendrückbaren Polyurethanschaum und eine Deckschicht aufweist, wobei ausgehend vom Rand der Wundauflage mindestens ein Einschnitt ausgebildet ist, der die Wundauflage teilweise trennt.

Ferner beschreibt US 2004/249328 A1 eine Wundauflage, die flächig ausgebildet ist und einen umlaufenden Rand aufweist, wobei die Wundauflage eine absorbierende Hydrogelschicht und eine Klebeschicht aufweist und ausgehend vom Rand der Wundauflage mindestens ein Einschnitt ausgebildet ist, der die Wundauflage teilweise trennt.

Eine flächige Wundauflage mit umlaufenden Rand und mindestens einem Einschnitt, der ausgehend vom Rand die Wundauflage teilweise trennt, wird auch in der US 2010/033175 A1 sowie in der US 2012/0004515 A1 beschrieben. Diese Wundauflagen umfassen Absorptionskörper, welche Superabsorber-Partikel enthalten können.

US-Patent 4 617 326 beschreibt eine Zusammensetzung mit einer hydrophoben antimikrobiellen Komponente und einer hydrophilen absorbierende Komponente.

Der vorliegenden Erfindung hat somit die Aufgabe zugrunde gelegen, den Einsatzbereich von Wundauflagen, insbesondere Wundauflagen zur Umlage um in den Körper hinein- oder aus dem Körper herausführende Leitungen und Schläuche, wie beispielsweise Katheter, zu erweitern und zu verbessern. Durch die erfindungsgemäße Wundauflage soll insbesondere eine sichere Fixierung der Leitungen und/oder Schläuche bewirkt werden. Des Weiteren soll die Wundöffnung möglichst dicht abgedeckt werden und die Tragedauer der Wundauflagen im Vergleich zu den bekannten Wundabdeckungen erhöht werden. Außerdem soll die Wundauflage antimikrobiell wirken, um Infektionen zu verhindern, und sich gut der Anatomie des Patienten anpassen, um leicht angelegt werden zu können.

Es hat sich nun herausgestellt, dass diese Aufgaben durch eine Wundauflage mit den Merkmalen des vorgelegten Hauptanspruchs gelöst werden. Gegenstand der Erfindung ist eine mehrschichtige Wundauflage, die flächig ausgebildet ist und einen umlaufenden Rand aufweist. Die Wundauflage umfasst
(a) mindestens eine hydrophobe antimikrobielle Schicht,
(b) mindestens eine absorbierende Schicht, die ein wasserhaltiges Hydrogel umfasst, und
(c) eine Deckschicht.

Des Weiteren ist ausgehend vom umlaufenden Rand der Wundauflage mindestens ein Einschnitt ausgebildet, der die Wundauflage teilweise trennt. Die erfindungsgemäße Wundauflage ist dadurch ausgezeichnet, dass sich die absorbierende Schicht (b) unmittelbar bis an den mindestens einen Einschnitt erstreckt, wobei sich die absorbierende Schicht (b) über die im Wesentlichen gesamte antimikrobielle Schicht (a) erstreckt und diese teilweise oder über den gesamten Querschnitt durchdringt.

Der Einschnitt und somit die teilweise Trennung der Wundauflage erfolgt insbesondere im Wesentlichen senkrecht zu den Schichten (a) bis (c). Weiterhin ist es besonders bevorzugt, dass die Schichten (a) bis (c) sowie ggf. weitere Schichten, die im Folgenden beschrieben werden, im Wesentlichen parallel übereinander angeordnet sind.

Gemäß einer ersten bevorzugten Ausführungsform der Erfindung ist die Wundauflage dadurch gekennzeichnet, dass die durch den mindestens einen Einschnitt getrennten Kanten der Wundauflage zumindest entlang eines Teils der Länge des Einschnitts unmittelbar aneinander anliegen. Besonders bevorzugt ist es, wenn die durch den Einschnitt getrennten Kanten der Wundauflage entlang der gesamten Länge des Einschnitts unmittelbar aneinander anliegen.

Gemäß einer anderen bevorzugten Ausführungsform der Erfindung ist die Wundauflage dadurch gekennzeichnet, dass der mindestens eine Einschnitt als längliche Aussparung mit beabstandet zueinander verlaufenden Kanten ausgebildet ist. In dieser Ausführungsform bildet der Einschnitt somit eine längliche Aussparung wie beispielsweise eine U-förmige Aussparung.

Gemäß einer Weiterbildung kann auch vorgesehen sein, dass der Einschnitt der Wundauflage eine oder mehrere Öffnungen wie z.B. Löcher aufweist, die zwischen dem Rand und dem vom Rand beabstandeten Ende des Einschnitts angeordnet sind. Diese Öffnungen können insbesondere kreisrund oder ellipsenförmig ausgebildet sein und können einen Durchmesser von 0,5 bis 10 mm, insbesondere 0,5 bis 5 mm aufweisen. Besonders bevorzugt erstreckt sich die eine oder die mehreren Öffnungen durch sämtliche Schichten der Wundauflage. Die Öffnungen dienen dazu, dass die Wundauflage leichter an den Querschnitt des zu umlegenden, in den Körper hinein- oder aus dem Körper herausführenden Schlauches angepasst werden kann.

In einer Ausführungsform dieser Weiterbildung weist der mindestens eine Einschnitt der Wundauflage zwischen dem umlaufenden Rand und dessen vom Rand entfernten Ende mindestens eine Öffnung auf. Beispielsweise befindet sich diese Öffnung etwa auf der Hälfte zwischen dem Rand und dem vom Rand entfernten Ende des Einschnitts.

Bevorzugt ist jedoch eine Wundauflage, bei der sich eine Öffnung am vom Rand entfernten Ende des Einschnitts befindet. Eine bevorzugte Ausführungsform der Erfindung betrifft somit eine Wundauflage, bei der der Einschnitt am vom Rand entfernten Ende mindestens eine Öffnung aufweist.

Gemäß einer weiteren Ausführungsform ist es bevorzugt, dass die Wundauflage anstelle der mindestens einen Öffnung oder zusätzlich zu der mindestens einen Öffnung Querschlitze aufweist, die quer zu dem mindestens einen Einschnitt verlaufen. Derartige Querschlitze befinden sich insbesondere in einem Hauptabschnitt des Einschnitts, der sich vom Rand bis zu einem vom Rand entfernten Ende erstreckt. Mithin ist die erfindungsgemäße Wundauflage in einer Ausführungsform dadurch gekennzeichnet, dass der mindestens eine Einschnitt einen Hauptabschnitt aufweist, der sich vom Rand bis zu einem vom Rand entfernten Ende erstreckt, und der Hauptabschnitt einen oder mehrere quer zum Einschnitt verlaufende Querschlitze aufweist. Ganz besonders bevorzugt bei dieser Ausführungsform ist es, wenn sich der eine oder die mehreren quer zum Einschnitt verlaufenden Querschlitze gleichermaßen auf beiden Seiten des Einschnitts erstrecken. Der Einschnitt und der eine oder die mehreren quer zum Einschnitt verlaufenden Querschlitze können zusammen somit ein oder mehrere kreuzförmige Schnitte bilden.

Gemäß einer weiteren besonders bevorzugten Ausführungsform der erfindungsgemäßen Wundauflage weist der mindestens eine Einschnitt der Wundauflage einen Hauptabschnitt auf, der sich vom Rand bis zu einem vom Rand entfernten Ende erstreckt, wobei sich am vom Rand entfernten Ende des Hauptabschnitts weg eine Vielzahl von Schlitzen erstreckt. Diese Vielzahl von Schlitzen können zusammen mit dem Hauptabschnitt beispielsweise Y-förmige, T-förmige, sternförmige oder kreuzförmige Schnitte bilden.

Die Hauptaufgabe des Einschnitts der Wundauflage sowie der gegebenenfalls vorhandenen oben diskutierten Öffnungen, Querschlitze und Vielzahl von Schlitzen am vom Rand entfernten Ende des Einschnitts ist es, eine gute Anpassung an den beispielsweise Katheterschlauch sowie an die anatomische Situation des Patienten zu erreichen, sodass die Wundauflage leicht um die in den Körper hinein- und aus dem Körper herausführenden Leitungen und Schläuche herumgelegt werden kann und sich gut der Topographie des Patienten anpasst.

Die Wundauflage ist dadurch gekennzeichnet, dass sich die absorbierende Schicht (b) unmittelbar bis an den mindestens einen Einschnitt erstreckt. Erfindungsgemäß liegen die durch den Einschnitt getrennten Kanten der absorbierenden Schicht (b) somit zumindest entlang eines Teils der Länge des Einschnitts, vorzugsweise entlang der im Wesentlichen gesamten Länge des Einschnitts, unmittelbar aneinander.

Bei der Schicht (a) der erfindungsgemäßen Wundauflage handelt es sich um eine hydrophobe antimikrobielle Schicht. Insbesondere umfasst die Schicht (a) ein Celluloseacetatgewebe, Viskosegewebe, Baumwollgewebe oder ein Mischgewebe, wobei als Mischgewebe vorzugsweise Schmelzklebfasern verwendet werden können. Besonders bevorzugt ist eine Schicht (a) auf Basis von Celluloseacetatgewebe und Baumwollgewebe, insbesondere Celluloseacetatgewebe. Die Hydrophobierung der hydrophoben antimikrobiellen Schicht (a) kann in einer Behandlung mit Dialkylcarbamoylchlorid (DACC) und/oder Alken-Keten-Dimer bestehen. In einer besonders bevorzugten Ausführungsform umfasst die Schicht (a) Celluloseacetatgewebe, das mit DAAC, wie Dihexadecylcarbamoylchlorid oder Dioctadecyclcarbamoylchlorid, beschichtet ist.

Die hydrophobe antimikrobielle Schicht (a) weist vorzugsweise ein flächenspezifisches Gewicht von 40 bis 80 g/m², insbesondere 54 bis 66 g/m² und besonders bevorzugt etwa 60 g/m² auf. Des Weiteren beträgt die Dicke der Schicht (a) vorzugsweise 0,2 bis 1,0 mm.

Bei der Schicht (b) der erfindungsgemäßen Wundauflage handelt es sich um eine absorbierende Schicht, die ein wasserhaltiges Hydrogel umfasst. In einer besonderen Ausführungsform umfasst das Hydrogel sog. superabsorbierende Polymere und insbesondere Polymere, die Hydroxy-, Carboxy-, Carboxamido-, Sulfonyl- und/oder Estergruppen aufweisen.

Insbesondere können als Polymere Superabsorber auf Methacrylsäure- oder Acrylsäurebasis, Polyvinylalkohol-Maleinsäureanhydrid-Copolymere, Polysaccharid-Maleinsäureanhydrid-Copolyere, Maleinsäurederivate, Acrylamidpropansulfonsäure-Copolymere, Stärke-Acrylnitril-Propfpolymere, gelatinisierte Stärkederivate, Alkyl- oder Hydroxyalkylcellulose, Carboxymethylcellulose, Stärke-Acrylsäure-Pfropfpolymere, Vinylacetat-Acrylsäureester-Copolymere, Acrylnitril- oder Acrylamid-Copolymere verwendet werden.

Bei dem in Schicht (b) verwendeten Hydrogel handelt es sich insbesondere um ein wasserunlösliches Polymer, dessen Moleküle durch ionische Bindungen zu einem dreidimensionalen Netzwerk verknüpft sind und das bei Kontakt mit Wasser unter Volumenzunahme quillt, ohne aber den stofflichen Zusammenhalt zu verlieren. Mithin ist das Hydrogel der Schicht (b) in der Lage, ein Vielfaches seines Eigengewichts an Flüssigkeiten wie Wasser oder wässrigen Salzlösungen, einschließlich Wundexsudat, aufzunehmen.

Insbesondere umfasst die Schicht (b) mindestens ein hydrophiles Polymer oder Copolymer, das aus von Acrylsäure abgeleiteten Monomeren bebildet wird. Die hydrophilen Polymere oder Copolymere weisen bevorzugt Sulfonyl-Seitengruppen auf. Ferner ist es bevorzugt, dass mindestens ein Teil dieser Sulfonyl-Seitengruppen in ihrer Salzform vorliegen, sodass von H⁺ verschiedene Gegenkationen in dem Hydrogel enthalten sind.

Besonders bevorzugt enthält das Hydrogel der Schicht (b) ein Polymer oder Coplymer, das aus Monomeren ausgewählt aus der Gruppe bestehend aus 2-Acrylamido-2-methylpropansulfonsäure-Natrium (NaAMPS), 2-Acrylamido-2-methylpropansulfonsäure-Kalium (AMPS-K), 2-Acrylamido-2-methylpropansulfonsäure-Ammonium (Ammonium-AMPS), Kalium-3-sulfonatopropylacrylat (SPAK), Natrium-3-sulfonatopropylacrylat (NaSPA), Acrylsäure oder Kombinationen davon gebildet ist. Am meisten bevorzugt enthält das Hydrogel der Schicht (b) ein Copolymer, das aus einer Kombination von (i) NaSPA und NaAMPS, (ii) SPAK und NaAMPS, (iii) NaAMPS und AMPS-K, (iv) NaAMPS und Ammonium-AMPS oder (v) NaAMPS und Acrylsäure gebildet ist.

Das Hydrogel der Schicht (b) kann ferner ein Vernetzungsmittel wie z.B. Tripropylenglycoldiacrylat enthalten. Die Menge des Vernetzungsmittels kann üblicherweise etwa 0,01 bis etwa 0,5 Gew.-% betragen, bezogen auf das Gewicht der hydrophilen Polymere oder Coplymere der Schicht (b). Der Vernetzungsgrad sollte jedoch nicht zu hoch sein, um die Gefahr eines Aufbrechens des Hydrogels bei Quellung zu reduzieren.

Der Wassergehalt der Schicht (b) beträgt vorzugsweise mindestens 20 Gew.-%, insbesondere mindestens 25 Gew.-% und ganz besonders bevorzugt etwa 30 Gew.-%, bezogen auf das Gesamtgewicht der Schicht (b).

Zur Erhöhung der Festigkeit und mechanischen Stabilität sowie zur Verbesserung der Handhabung der Schicht (b) und somit der erfindungsgemäßen Wundauflage kann die absorbierende Schicht (b) in einer weiteren Ausführungsform eine Stabilisierungsstruktur ausgewählt aus der Gruppe bestehend aus einem Gewebe, einem Vlies, einem Gitter, einem Netz oder Kombinationen davon umfassen. Vorzugsweise umfasst Schicht (b) als Stabilisierungsstruktur ein Polyestergewebe, insbesondere ein Polyestergewebe mit einem flächenspezifischen Gewicht von etwa 10 bis 25 g/m², besonders bevorzugt etwa 18 g/m². Die Position der Stabilisierungsstruktur kann senkrecht zur Schichtebene der erfindungsgemäßen Wundauflage beliebig gewählt werden. Die Stabilisierungsstruktur kann im Inneren der absorbierenden Schicht (b) angeordnet sein, d.h. in der Schicht (b) eingebettet sein, oder auf eine der Außenflächen der Schicht (b) aufgebracht sein.

Die absorbierende Schicht (b) weist beispielsweise ein flächenspezifisches Gewicht von 1,7 kg/m² bis 2,1 kg/m² auf.

Der umlaufende Rand der absorbierenden Schicht (b) liegt in einer Ausführungsform unmittelbar an dem umlaufenden Rand der gesamten Wundauflage an. In einer bevorzugten Ausführungsform ist der umlaufende Rand der absorbierenden Schicht (b) jedoch beabstandet zu dem umlaufenden Rand der Wundauflage ausgebildet. D.h. in Draufsicht ist die Fläche der absorbierenden Schicht (b) kleiner als die Fläche der gesamten Wundauflage und die absorbierende Schicht befindet sich in einem zentralen Bereich der Wundauflage. Ein Randbereich der Wundauflage ist in dieser Ausführungsform somit frei von absorbierender Schicht (b).

Die Deckschicht (c) der erfindungsgemäßen Wundauflage umfasst vorzugsweise eine Polyurethanfolie. Diese Polyurethanfolie kann wasserundurchlässig oder wasserdurchlässig ausgebildet sein und ist insbesondere wasserdurchlässig. Die Dicke der Deckschicht (c) beträgt vorzugsweise 10 bis 70 µm, vorzugsweise 30 bis 50 µm und besonders bevorzugt etwa 30 µm.

Zusätzlich zu den Schichten (a) bis (c) kann die erfindungsgemäße Wundauflage weitere Schichten aufweisen.

In einer Ausführungsform weist die Wundauflage eine Klebeschicht auf. Falls vorhanden, erstreckt sich die Klebeschicht in Form eines Klebestreifens entlang des Randbereichs der Wundauflage und ist auf der von der absorbierenden Schicht (b) wegweisenden Seite der antimikrobiellen Schicht (a) aufgebracht, wobei der Klebestreifen die antimikrobielle Schicht nur teilweise abdeckt. Auf diese Weise wird ein Verkleben der Wundöffnung des Patienten, die üblicherweise durch einen zentralen Bereich der Wundauflage abgedeckt wird, mit der Klebeschicht und somit ein ungünstiges Wundklima vermieden.

Die Klebeschicht kann beispielsweise durch Verwendung eines Polyacrylatklebers, eines Silikonklebers oder eines Hydrokolloidklebers bereitgestellt werden. Besonders bevorzugt enthält die Klebeschicht ein wasserhaltiges Hydrogel. Grundsätzlich eignen sich die oben im Zusammenhang mit der absorbierenden Schicht (b) beschriebenen Hydrogele auch zur Verwendung als Hydrogel in der Klebeschicht. Ganz besonders bevorzugt enthält die Klebeschicht ein ionisches Copolymer aus Acrylsäure und einem Salz der Acrylsäure, wobei der Gehalt an Acrylsäure-Einheiten etwa 2 Gew.-% beträgt.

In einer weiteren Ausführungsform kann die Klebeschicht eine Stabilisierungsstruktur ausgewählt aus der Gruppe bestehend aus einem Gewebe, einem Vlies, einem Gitter, einem Netz oder Kombinationen davon umfassen. Vorzugsweise umfasst die Klebeschicht als Stabilisierungsstruktur ein Polypropylengewebe, vorzugsweise ein Polypropylengewebe mit einem flächenspezifischen Gewicht von etwa 15 bis 25 g/m², besonders bevorzugt etwa 20 g/m². Die Stabilisierungsstruktur ist vorzugsweise in das Innere der Klebeschicht eingebettet und die Position der Stabilisierungsstruktur kann senkrecht zur Schichtebene der erfindungsgemäßen Wundauflage innerhalb der Klebeschicht beliebig gewählt werden.

Gemäß einer alternativen Ausführungsform weist die erfindungsgemäße Wundauflage keine separat ausgestaltete Klebeschicht auf. Denn erfindungsgemäß erstreckt sich die absorbierende Schicht (b) über im Wesentlichen die gesamte antimikrobielle Schicht (a), d.h. über einen zentralen Bereich und den Randbereich der Wundauflage. Dabei durchdringt das Material der absorbierenden Schicht (b) die antimikrobielle Schicht (a) teilweise oder vollständig. Auf diese Weise kann die absorbierende Schicht bei der Anwendung der Wundauflage eine Klebewirkung erzielen und für eine sichere und zuverlässige Befestigung der Wundauflage beim Patienten sorgen. Insbesondere wird die antimikrobielle Schicht (a) nur teilweise, vorzugsweise in einem Randbereich der Wundauflage, von der absorbierenden Schicht (b) durchdrungen, so dass ein Verkleben der Wundöffnung des Patienten, die üblicherweise durch einen zentralen Bereich der Wundauflage abgedeckt wird, mit als Klebeschicht fungierenden absorbierenden Schicht (b) und somit ein ungünstiges Wundklima vermieden wird. Gemäß dieser erfindungsgemäßen Ausführungsform, bei der sich die absorbierende Schicht (b) über im Wesentlichen die gesamte antimikrobielle Schicht (a) erstreckt und diese teilweise oder über den gesamten Querschnitt durchdringt, ist die Dicke der absorbierenden Schicht (b) üblicherweise etwas geringer im Vergleich zu einer nicht erfindungsgemäßen Ausführungsform, bei der sich die absorbierende Schicht nur über einen zentralen Bereich der Wundauflage erstreckt und im Randbereich eine separate Klebeschicht ausgebildet ist.

Zusätzlich zu den oben genannten Schichten kann die Wundauflage eine abziehbare Schutzschicht aufweisen. Diese grenzt zweckmäßigerweise an die hydrophobe antimikrobielle Schicht (a) oder, falls eine Klebeschicht vorhanden ist, an die Klebeschicht. Die abziehbare Schutzschicht umfasst vorzugsweise silikonisiertes Papier oder ein geeignetes Polymer wie ein silikonisierter Polyester, z.B. silikonisiertes Polyethylenterephthalat. Die abziehbare Schutzschicht wird zweckmäßigerweise vor Anwendung der Wundauflage am Patienten entfernt.

In einer besonders bevorzugten Ausführungsform weist die erfindungsgemäße Wundauflage somit folgende Schichten in folgender Reihenfolge auf:
- eine Deckschicht (c), vorzugsweise eine wasserdurchlässige Polyurethanfolie,
- eine absorbierende Schicht (b), die mindestens ein wasserhaltiges Hydrogel umfasst, vorzugsweise ein Copolymer auf Basis von Acrylsäuremonomeren mit Sulfonylseitengruppen mit einem Wassergehalt von mindestens 20 Gew.-%, wobei im Inneren des Hydrogels als Stabilisierungsstruktur, vorzugsweise ein Polyestergewebe mit einem flächenspezifischen Gewicht von etwa 10 bis 25 g/m², eingebettet ist,

- eine hydrophobe antimikrobielle Schicht (a), vorzugsweise eine Celluloseacetatgewebe, das mit DAAC beschichtet ist,
- ggf. eine Klebeschicht, die vorzugsweise ein wasserhaltiges Hydrogel wie ein Copolymer auf Basis von Acrylsäuremonomeren mit einem Wassergehalt von mindestens 20 Gew.-% umfasst, wobei im Inneren der Klebeschicht als Stabilisierungsstruktur vorzugsweise ein Polypropylengewebe mit einem flächenspezifischen Gewicht von etwa 15 bis 25 g/m² eingebettet ist, und
- ggf. eine abziehbare Schutzschicht, die vorzugsweise ein silikonisiertes Polyethylenterephthalat umfasst.

Die Wundauflage kann in Draufsicht eine rotationssymmetrische Grundform, beispielsweise die Form eines Kreises, einer Ellipse, eines Quadrats oder eines regelmäßigen Vielecks, oder jede andere Form aufweisen. In einer Ausführungsform, die besonders für eine Anwendung der Wundauflage im Bereich des Kreuzbeins von Patienten geeignet ist, weist die erfindungsgemäße Wundauflage in Draufsicht eine herzförmige Grundform auf.

Es hat sich herausgestellt, dass sich die erfindungsgemäße Wundauflage durch ihren speziellen Aufbau besonders gut um aus dem Körper heraus- oder in den Körper hineinführende Leitungen und Schläuche gelegt werden kann. Ein besonderer Vorteil der erfindungsgemäßen Wundauflage besteht außerdem darin, dass sie durch Quellung des Hydrogels der absorbierenden Schicht (b) aufgrund von Exsudataufnahme in der Lage ist, die Wundöffnung weitestgehend vollständig abzudecken und somit den Zugang zum Körper für z.B. Bakterien sicher zu verschließen. Dieser Vorteil wird insbesondere dadurch erreicht, dass sich die absorbierende Schicht (b) unmittelbar bis an den Einschnitt der Wundauflage erstreckt. Durch die Quellung der absorbierenden Schicht (b) wird eine zusätzliche Stabilisierung des z.B. Katheters erreicht und es herrschen geringe Scherkräfte innerhalb der Wundauflage sowie in Bezug auf eine zu behandelnde Wunde.

Im Folgenden wird die vorliegende Erfindung anhand von Zeichnungen erläutert, wobei die folgenden Figuren 1 bis 13 nicht erfindungsgemäße Ausgestaltungen zeigen, die lediglich zur Veranschaulichung dienen, wobei
- Fig. 1: eine Draufsicht auf eine erste Ausgestaltung zeigt,
- Fig. 2: einen Schnitt entlang der Linie II-II aus Figur 1 zeigt,
- Fig. 3: eine Draufsicht auf eine zweite Ausgestaltung zeigt,
- Fig. 4: eine Draufsicht auf eine dritte Ausgestaltung zeigt,
- Fig. 5: eine Draufsicht auf eine vierte Ausgestaltung zeigt,
- Fig. 6: eine Draufsicht auf eine fünfte Ausgestaltung zeigt,
- Fig. 7: eine Draufsicht auf eine sechste Ausgestaltung zeigt,
- Fig. 8: eine Draufsicht auf eine siebte Ausgestaltung zeigt,
- Fig. 9: eine Draufsicht auf eine achte Ausgestaltung zeigt,
- Fig. 10: eine Draufsicht auf eine neunte Ausgestaltung zeigt,
- Fig. 11: eine Draufsicht auf eine zehnte Ausgestaltung zeigt,
- Fig. 12: eine Draufsicht auf eine elfte Ausgestaltung zeigt,
- Fig. 13: eine Draufsicht auf eine zwölfte Ausgestaltung zeigt,
- Fig. 14: eine Draufsicht auf eine dreizehnte bevorzugte Ausgestaltung zeigt und
- Fig. 15: einen Schnitt entlang der Linie II-II aus Figur 14 zeigt.

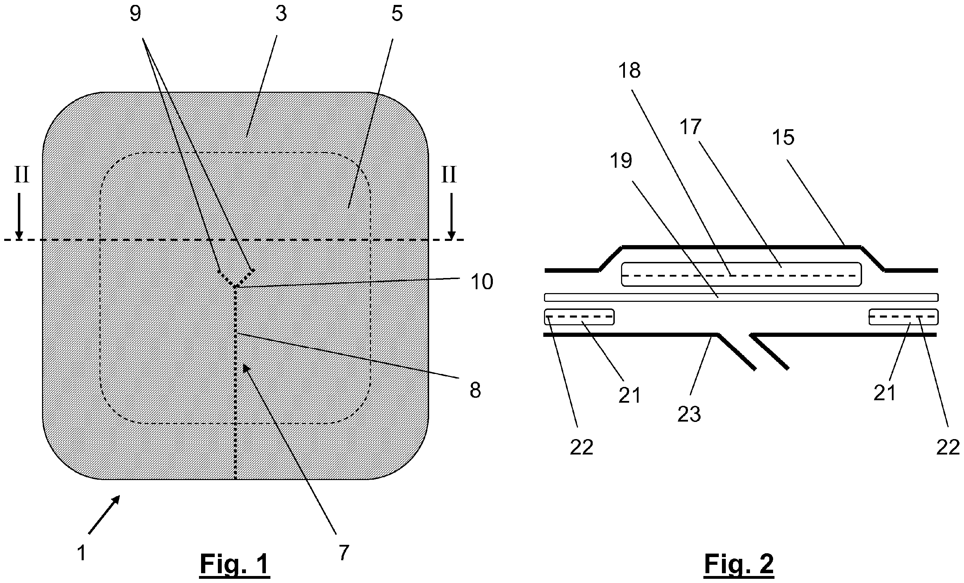

Figur 1 zeigt eine Wundauflage 1 mit einem Randbereich 3 und einem zentralen Bereich 5. Ferner weist die Wundauflage einen Einschnitt 7 auf. Der Einschnitt 7 weist einen Hauptabschnitt 8 auf, der sich vom Rand weg erstreckt. Zwei Schlitze 9 erstrecken sich vom vom Rand entfernten Ende 10 des Hauptabschnitts 8 weg, die zusammen mit dem Hauptabschnitt 8 einen Y-förmigen Einschnitt 7 bilden und somit zusätzlich zu dem Hauptabschnitt 8 eine bessere Anpassung der erfindungsgemäßen Wundauflage 1 um z.B. einen Katheterschlauch herum ermöglichen. In einer nicht dargestellten Ausgestaltung der Wundauflage sind die Schlitze 9 am vom Rand entfernten Ende 10 des Hauptabschnitts 8 nicht vorhanden, sodass die Wundauflage lediglich einen geraden Einschnitt 7 aufweist.

Figur 2 zeigt einen Querschnitt der Wundauflage 1 gemäß Figur 1 entlang der Linie II-II. Die Wundauflage 1 weist als oberste Schicht eine Deckschicht (c) 15 auf, die sich über den gesamten Querschnitt erstreckt. Unterhalb der Deckschicht 15 ist im zentralen Bereich 5 der Wundauflage 1 eine absorbierende Schicht (b) 17 ausgestaltet, die ein wasserhaltiges Hydrogel umfasst. In die absorbierende Schicht (b) 17 ist eine Stabilisierungsstruktur 18 eingebettet, die zur Erhöhung der Festigkeit und einer Verbesserung der Handhabung der Wundauflage führt. Der Randbereich 3 der Wundauflage 1 ist frei von der absorbierenden Schicht 17. Unterhalb der absorbierenden Schicht 17 ist eine hydrophobe antimikrobielle Schicht (a) 19 ausgestaltet, die sich über den gesamten Querschnitt der Wundauflage 1 erstreckt. Zur Befestigung der Wundauflage 1 am Patienten ist im Randbereich 3 der Wundauflage 1 eine Klebeschicht 21 in Form eines Klebestreifens ausgebildet, in die eine Stabilisierungsstruktur 22 eingebettet ist. Des Weiteren weist die Wundauflage 1 gemäß Figur 1 eine abziehbare Schutzschicht 23 auf, die auf der von der Schicht (a) 19 wegweisenden Seite der Klebeschicht 21 aufgebracht ist.

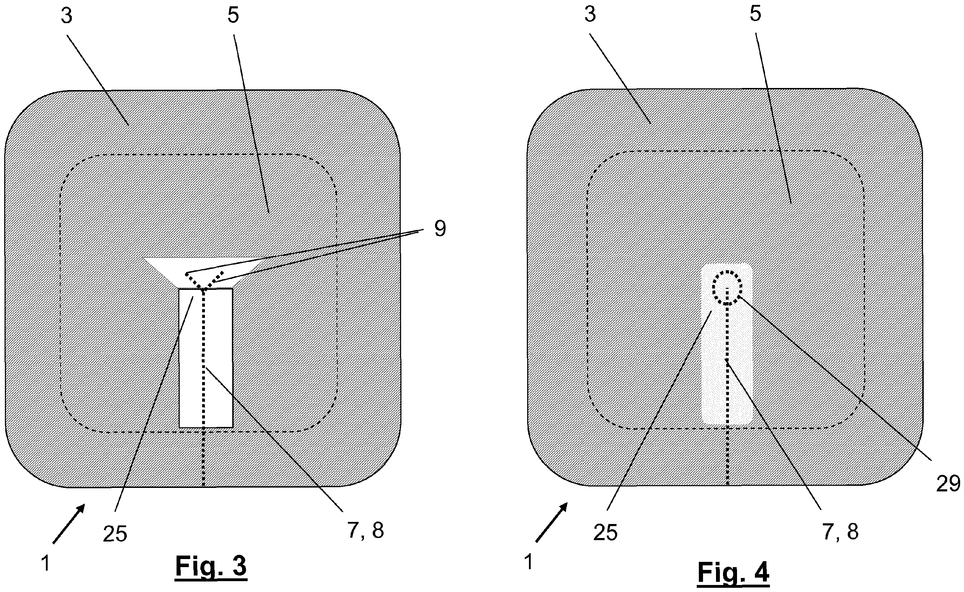

Die in den folgenden Figuren 3 bis 13 gezeigten Wundauflagen weisen im Wesentlichen den gleichen Schichtaufbau der Wundauflage 1 auf, wie er in Figur 2 gezeigt ist.

Figur 3 zeigt eine Wundauflage 1, die im Wesentlichen der Ausgestaltung der in Figur 1 gezeigten Wundauflage 1 entspricht. Im Unterschied zur Wundauflage 1 gemäß Figur 1 weist die Wundauflage 1 gemäß Figur 3 einen Bereich 25 auf, der frei von absorbierender Schicht 17 ist. Die absorbierende Schicht 17 ist somit im Bereich 25 ausgespart und die an den Bereich 25 angrenzenden Kanten der Schicht 17 sind beabstandet zueinander ausgebildet. Der Einschnitt 7 sowie die Schlitze 9 der Wundauflage 1 befinden sich in dem Bereich 25. Die hydrogelhaltige absorbierende Schicht 17 reicht somit nicht an den Einschnitt 7 und die Schlitze 9 heran.

Figur 4 zeigt eine Wundauflage 1, bei der am vom Rand entfernten Ende des Einschnitts 7 eine kreisrunde Öffnung 29 ausgebildet ist. Durch die kreisrunde Öffnung 29 kann die Wundauflage 1 noch leichter an die Anatomie des Patienten und die Topographie der in den Körper hinein oder aus dem Körper hinaus führenden Leitungen und Schläuche angepasst werden. Der Durchmesser der kreisrunden Öffnung 29 hängt insbesondere von dem Durchmesser der beim Patienten verwendeten Leitungen und Schläuche, wie z.B. Katheter, ab.

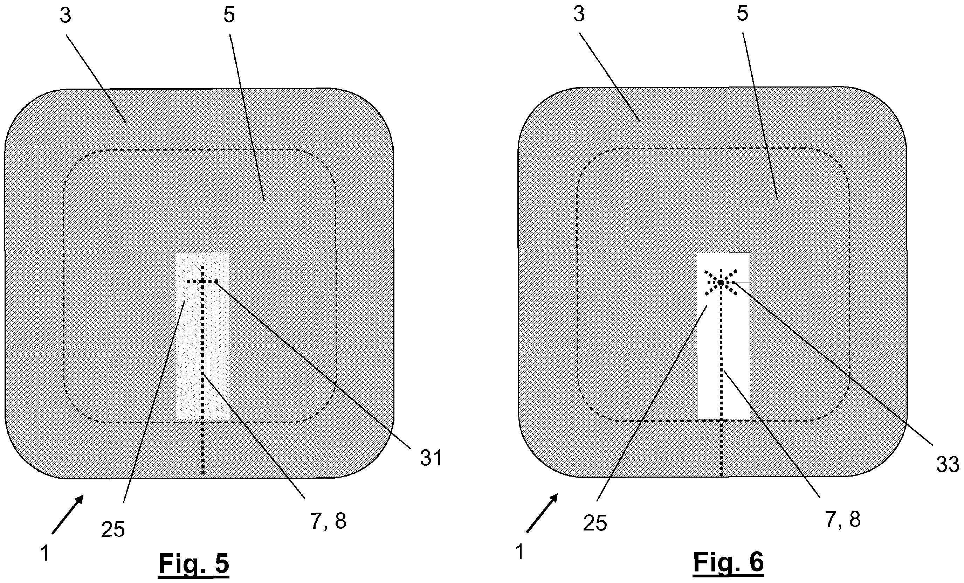

Figur 5 zeigt eine Wundauflage 1 mit einem Bereich 25, in dem die absorbierende Schicht 17 ausgespart ist. Am vom Rand entfernten Ende des Hauptabschnitts 8 sind drei Schlitze 31 ausgebildet, die zusammen mit dem Haupabschnitt 8 einen kreuzförmigen Einschnitt 7 bilden.

Figur 6 zeigt eine Wundauflage 1, die der Wundauflage gemäß Figur 5 entspricht. Anstelle der kreuzförmig angeordneten Schlitze 31 weist die Wundauflage 1 gemäß Figur 6 sternförmige Schlitze 33 auf.

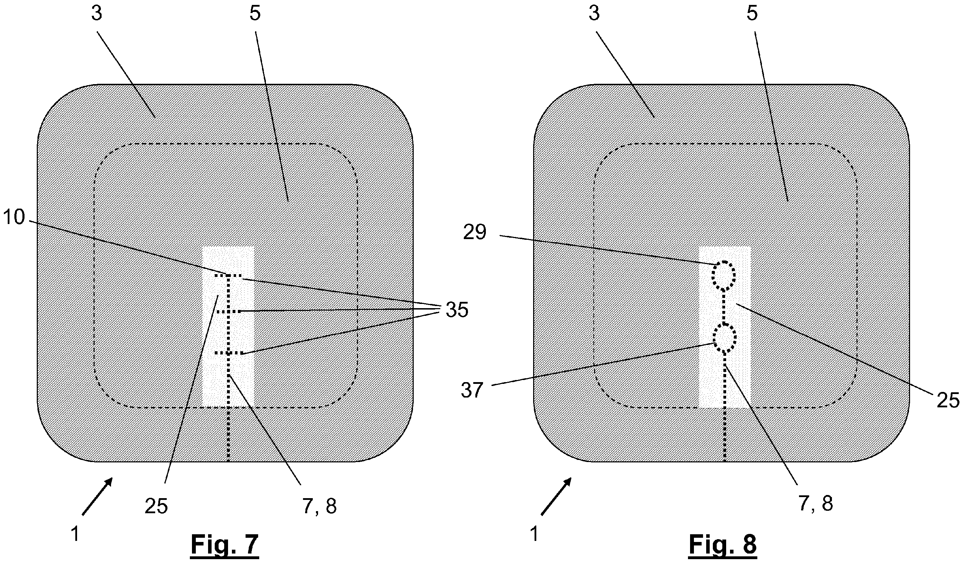

Figur 7 zeigt eine Wundauflage 1 mit einem Bereich 25, in dem die absorbierende Schicht 17 ausgespart ist. Der Einschnitt 7 weist einen Hauptabschnitt 8 auf, der sich vom Rand der Wundauflage 1 bis zu einem vom Rand entfernten Ende 10 erstreckt, wobei der Hauptabschnitt 8 mehrere quer zum Hauptabschnitt 8 verlaufende Querschlitze 35 aufweist.

Figur 8 zeigt eine Wundauflage 1 mit einem Bereich 25, in dem die absorbierende Schicht 17 ausgespart ist, wobei der Einschnitt 7 am vom Rand entfernten Ende eine Öffnung 29 sowie zwischen dem Rand und dem vom Rand entfernten Ende des Einschnitts 7 eine Öffnung 37 aufweist.

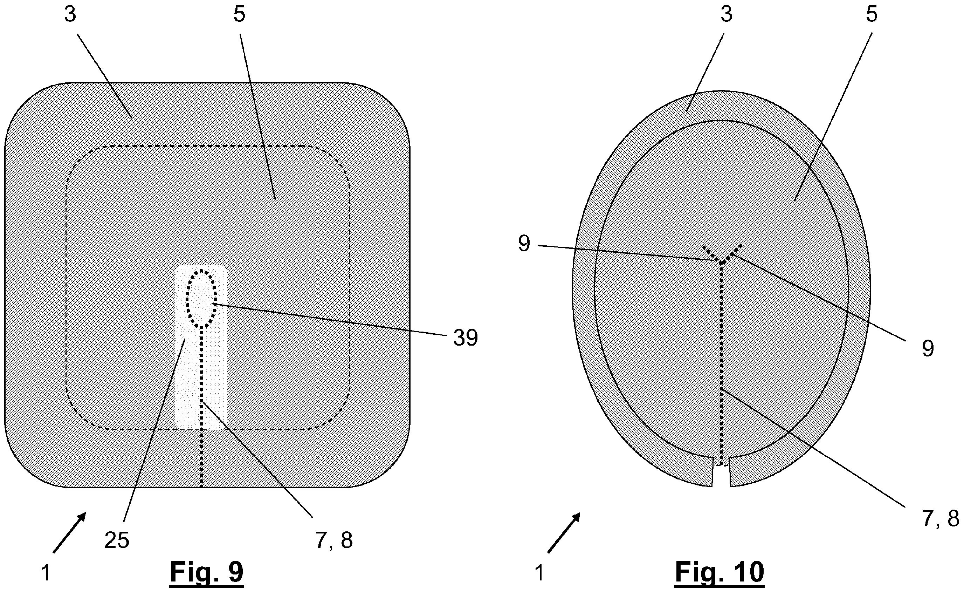

Figur 9 zeigt eine Wundauflage 1 mit einem Einschnitt 7 und einem Bereich 25, in dem die absorbierende Schicht 17 ausgespart ist, wobei am vom Rand entfernten Ende des Einschnitts 7 eine ellipsenförmige Öffnung 39 ausgebildet ist.

Figur 10 zeigt eine Wundauflage 1, die der Wundauflage 1 gemäß Figur 1 entspricht. Im Unterschied zu der Wundauflage 1 gemäß Figur 1 weist die Wundauflage 1 gemäß Figur 10 eine ellipsenförmige Grundfläche auf. Dementsprechend nimmt auch der zentrale Bereich 5 der Wundauflage 1 eine ellipsenförmige Gestalt an.

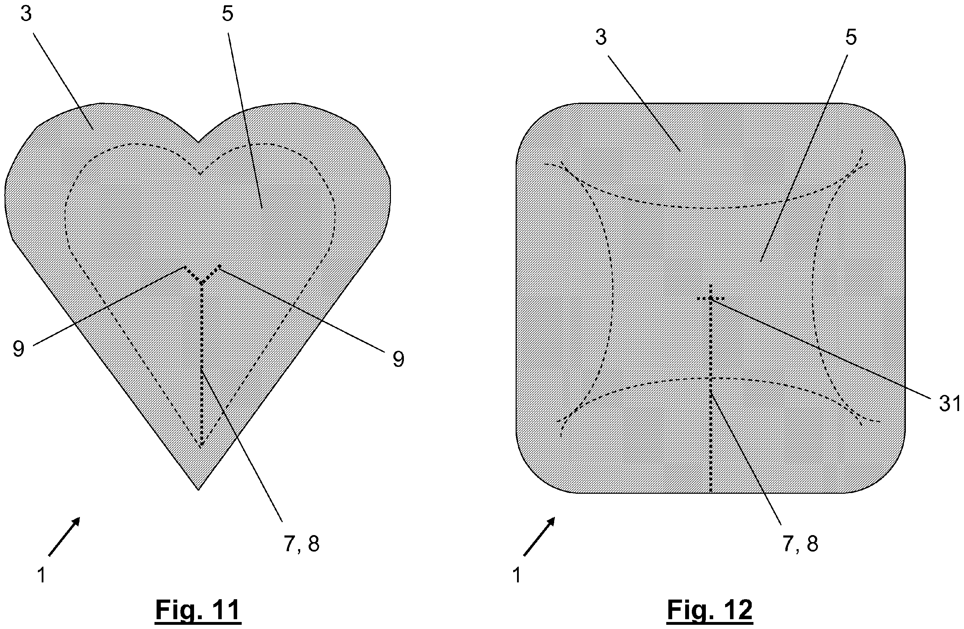

Figur 11 zeigt eine Wundauflage 1, die der Wundauflage 1 gemäß Figur 1 entspricht. Im Unterschied zu der Wundauflage 1 gemäß Figur 1 weist die Wundauflage 1 gemäß Figur 11 eine herzförmige Grundform auf, wodurch sie sich besonders zur Abdeckung von Wunden im Bereich des Kreuzbeins eignet.

Figur 12 zeigt eine Wundauflage 1 mit einem Einschnitt 7 und kreuzförmigen Schlitzen 31 am vom Rand entfernten Ende des Einschnitts 7. Der zentrale Bereich 5 der Wundauflage 1 ist sternenförmig ausgebildet, wodurch sich die Wundauflage besonders zur Abdeckung von Wunden im Bereich der Armbeuge oder des Gesäßes eignet.

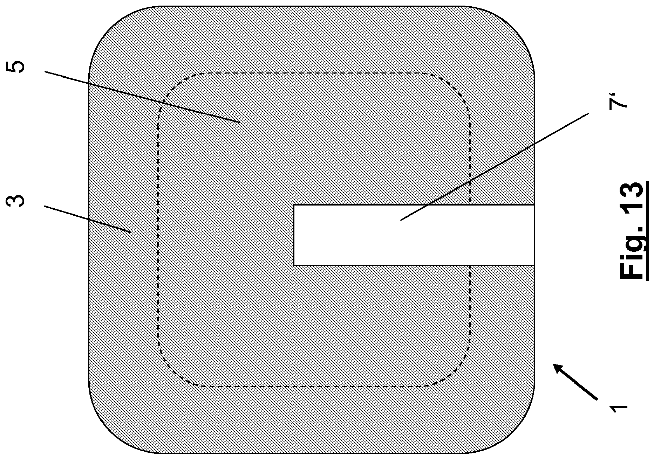

Figur 13 zeigt eine Wundauflage 1 mit einem Einschnitt 7', der als längliche Aussparung mit zwei beabstandet zueinander verlaufenden Kanten ausgebildet ist.

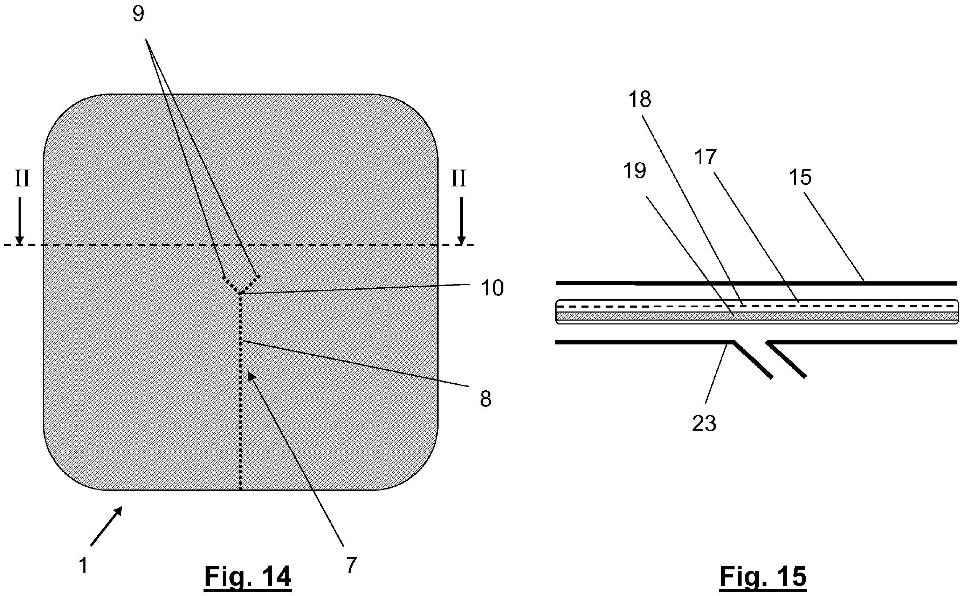

Figur 14 zeigt eine erfindungsgemäße Wundauflage 1 mit einem Einschnitt 7, der einen sich vom Rand weg erstreckenden Hauptabschnitt 8 aufweist. Zwei Schlitze 9 erstrecken sich vom vom Rand entfernten Ende 10 des Hauptabschnitts 8 weg und bilden zusammen mit dem Hauptabschnitt 8 einen Y-förmigen Einschnitt 7 bilden.

Figur 15 zeigt einen Querschnitt der erfindungsgemäßen Wundauflage 1 gemäß Figur 14 entlang der Linie II-II. Die Wundauflage 1 weist als oberste Schicht eine Deckschicht (c) 15 auf, die sich über den gesamten Querschnitt erstreckt. Unterhalb der Deckschicht 15 erstreckt sich über den gesamten Querschnitt der Wundauflage 1 eine absorbierende Schicht (b) 17, die ein wasserhaltiges Hydrogel umfasst. In die absorbierende Schicht (b) 17 ist eine Stabilisierungsstruktur 18 eingebettet, die zur Erhöhung der Festigkeit und einer Verbesserung der Handhabung der Wundauflage führt. Des Weiteren durchdringt die absorbierende Schicht (b) 17 zur Erzielung eines Klebeeffektes eine hydrophobe antimikrobielle Schicht (a) 19, die sich ebenfalls über den gesamten Querschnitt der Wundauflage 1 erstreckt. Außerdem weist die Wundauflage 1 gemäß Figur 14 eine abziehbare Schutzschicht 23 auf, die auf der von der Schicht (c) 15 wegweisenden Seite der absorbierenden Schicht 17 aufgebracht ist.

## Patentansprüche

1. Mehrschichtige Wundauflage (1), die flächig ausgebildet ist und einen umlaufenden Rand aufweist, umfassend
(a) mindestens eine hydrophobe antimikrobielle Schicht (19),
(b) mindestens eine absorbierende Schicht (17), die ein wasserhaltiges Hydrogel umfasst, und
(c) eine Deckschicht (15),
wobei ausgehend vom Rand der Wundauflage mindestens ein Einschnitt (7) ausgebildet ist, der die Wundauflage (1) teilweise trennt, **dadurch gekennzeichnet, dass** sich die absorbierende Schicht (b) (17) unmittelbar bis an den mindestens einen Einschnitt (7) erstreckt, wobei sich die absorbierende Schicht (b) (17) über die im Wesentlichen gesamte antimikrobielle Schicht (a) (19) erstreckt und diese teilweise oder über den gesamten Querschnitt durchdringt.

2. Wundauflage nach Anspruch 1, bei der die durch den mindestens einen Einschnitt (7) getrennten Kanten der Wundauflage zumindest entlang eines Teils der Länge des Einschnitts (7) unmittelbar aneinander anliegen.

3. Wundauflage nach Anspruch 1, bei der der mindestens eine Einschnitt (7') als längliche Aussparung mit beabstandet zueinander verlaufenden Kanten ausgebildet ist.

4. Wundauflage nach einem der vorhergehenden Ansprüche, wobei der mindestens eine Einschnitt (7) zwischen dem Rand und dessen vom Rand entfernten Ende (10) mindestens eine kreisrunde oder ellipsenförmige Öffnung aufweist.

5. Wundauflage nach einem der vorhergehenden Ansprüche, wobei der Einschnitt (7) am vom Rand entfernten Ende (10) mindestens eine kreisrunde oder ellipsenförmige Öffnung (29) aufweist.

6. Wundauflage nach einem der vorhergehenden Ansprüche, wobei der mindestens eine Einschnitt (7) einen Hauptabschnitt (8) aufweist, der sich vom Rand bis zu einem vom Rand entfernten Ende (10) erstreckt, und der Hauptabschnitt (8) einen oder mehrere quer zum Einschnitt (7) verlaufende Querschlitze (35) aufweist.

7. Wundauflage nach einem der vorhergehenden Ansprüche, wobei der mindestens eine Einschnitt (7) einen Hauptabschnitt (8) aufweist, der sich vom Rand bis zu einem vom Rand entfernten Ende (10) erstreckt, und sich vom vom Rand entfernten Ende (10) des Hauptabschnitts (8) weg eine Vielzahl von Schlitzen (9) erstreckt.

8. Wundauflage nach einem der vorhergehenden Ansprüche, bei der die hydrophobe antimikrobielle Schicht (a) (19) ein Celluloseacetatgewebe, Viskosegewebe, Baumwollgewebe oder ein Mischgewebe umfasst.

9. Wundauflage nach Anspruch 8, bei dem die Schicht (a) (19) mit Dialkylcarbamoylchlorid und/oder Alken-Keten-Dimer behandelt worden ist.

10. Wundauflage nach einem der vorhergehenden Ansprüche, bei der die absorbierende Schicht (b) (17) einen Wassergehalt von mindestens 20 Gew.-% aufweist.

11. Wundauflage nach einem der vorhergehenden Ansprüche, bei der die absorbierende Schicht (b) (17) ein Polymer oder Coplymer umfasst, das aus Monomeren ausgewählt aus der Gruppe bestehend aus 2-Acrylamido-2-methylpropansulfonsäure-Natrium (NaAMPS), 2-Acrylamido-2-methylpropansulfonsäure-Kalium (AMPS-K), 2-Acrylamido-2-methylpropansulfonsäure-Ammonium (Ammonium-AMPS), Kalium-3-sulfonatopropylacrylat (SPAK), Natrium-3-sulfonatopropylacrylat (NaSPA), Acrylsäure oder Kombinationen davon gebildet ist.

12. Wundauflage nach einem der vorhergehenden Ansprüche, bei der die Deckschicht (c) (15) eine Polyurethanfolie umfasst.

13. Wundauflage nach einem der vorhergehenden Ansprüche, die zusätzlich eine Klebeschicht (21) in Form eines Klebestreifens aufweist, der sich entlang des Randes der Wundauflage erstreckt und auf der von der absorbierenden Schicht (b) (17) wegweisenden Seite der antimikrobiellen Schicht (a) (19) aufgebracht ist, wobei der Klebestreifen die antimikrobielle Schicht (a) (19) nur teilweise abdeckt.

14. Wundauflage nach einem der vorhergehenden Ansprüche, die zusätzlich eine abziehbare Schutzschicht (23) aufweist.

## Claims

1. Multilayered wound dressing (1) which has a width and length and has a peripheral edge, comprising
(a) at least one hydrophobic antimicrobial layer (19),
(b) at least one absorbent layer (17) which comprises a water-containing hydrogel, and
(c) a covering layer (15),
wherein, starting from the edge of the wound dressing, at least one incision (7) is formed, which partially separates the wound dressing (1), **characterized in that** the absorbent layer (b) (17) extends directly to the at least one incision (7), wherein the absorbent layer (b) (17) extends over essentially the entire antimicrobial layer (a) (19) and penetrates this partially or over the entire cross-section.

2. Wound dressing according to claim 1, in which the edges of the wound dressing separated by the at least one incision (7) lie directly adjacent to one another at least along a part of the length of the incision (7).

3. Wound dressing according to claim 1, in which the at least one incision (7') is formed as an elongated recess having edges running at a distance from one another.

4. Wound dressing according to one of the preceding claims, wherein the at least one incision (7) has at least one circular or ellipsoidal opening between the edge and the end (10) thereof remote from the edge.

5. Wound dressing according to one of the preceding claims, wherein the incision (7) has at least one circular or ellipsoidal opening (29) at the end (10) remote from the edge.

6. Wound dressing according to one of the preceding claims, wherein the at least one incision (7) has a main section (8) which extends from the edge to an end (10) remote from the edge, and the main section (8) has one or more transverse slits (35) running transversely to the incision (7) .

7. Wound dressing according to one of the preceding claims, wherein the at least one incision (7) has a main section (8) which extends from the edge to an end (10) remote from the edge, and a multiplicity of slits (9) extends away from the end (10) of the main section (8) remote from the edge.

8. Wound dressing according to one of the preceding claims, in which the hydrophobic antimicrobial layer (a) (19) comprises a cellulose acetate fabric, viscose fabric, cotton fabric or a blended fabric.

9. Wound dressing according to claim 8, in which the layer (a) (19) has been treated with dialkylcarbamoyl chloride and/or alkene ketene dimer.

10. Wound dressing according to one of the preceding claims, in which the absorbent layer (b) (17) has a water content of at least 20 wt.%.

11. Wound dressing according to one of the preceding claims, in which the absorbent layer (b) (17) comprises a polymer or copolymer which is formed from monomers chosen from the group consisting of sodium 2-acrylamido-2-methylpropanesulphonate (NaAMPS), potassium 2-acrylamido-2-methylpropanesulphonate (AMPS-K), ammonium 2-acrylamido-2-methylpropanesulphonate (ammonium-AMPS), potassium 3-sulphonatopropylacrylate (SPAK), sodium 3-sulphonatopropylacrylate (NaSPA), acrylic acid or combinations thereof.

12. Wound dressing according to one of the preceding claims, in which the covering layer (c) (15) comprises a polyurethane film.

13. Wound dressing according to one of the preceding claims, which additionally has an adhesive layer (21) in the form of an adhesive strip which extends along the edge of the wound dressing and is applied to the side of the antimicrobial layer (a) (19) facing away from the absorbent layer (b) (17), the adhesive strip only partially covering the antimicrobial layer (a) (19).

14. Wound dressing according to one of the preceding claims, which additionally has a peelable protective layer (23).

## Revendications

1. Pansement multicouche (1) qui présente une forme plate avec un bord périphérique, comprenant
(a) au moins une couche hydrophobe antimicrobienne (19),
(b) au moins une couche absorbante (17) qui comprend un hydrogel contenant de l'eau et
(c) une couche de recouvrement (15),
dans lequel, à partir du bord du pansement, est réalisée au moins une entaille (7) qui sépare partiellement le pansement (1), **caractérisé en ce que** la couche absorbante (b) (17) s'étend directement jusqu'à l'au moins une entaille (7), dans lequel la couche absorbante (b) (17) s'étend globalement au-dessus toute la couche antimicrobienne (a) (19) et traverse celle-ci partiellement ou sur toute la section transversale.

2. Pansement selon la revendication 1, dans lequel les arêtes du pansement, séparées par l'au moins une entaille (7) s'appuient, au moins le long d'une partie de la longueur de l'entaille (7) directement l'une contre l'autre.

3. Pansement selon la revendication 1, dans lequel l'au moins une entaille (7') est conçue comme un évidement allongé avec des arêtes s'étendant de manière distante entre elles.

4. Pansement selon l'une des revendications précédentes, dans lequel l'au moins une entaille (7) comprend, entre le bord et son extrémité (10) éloignée du bord, au moins une ouverture circulaire ou elliptique.

5. Pansement selon l'une des revendications précédentes, dans lequel l'entaille (7) comprend, à l'extrémité (10) éloignée du bord, au moins une ouverture circulaire ou elliptique (29).

6. Pansement selon l'une des revendications précédentes, dans lequel l'au moins une entaille (7) comprend une partie principale (8) qui s'étend du bord jusqu'à une extrémité (10) éloignée du bord et la partie principale (8) comprend une ou plusieurs fentes transversales (35) s'étendant transversalement par rapport à l'entaille (7).

7. Pansement selon l'une des revendications précédentes, dans lequel l'au moins une entaille (7) comprend une partie principale (8) qui s'étend du bord jusqu'à une extrémité (10) éloignée du bord et, de l'extrémité (10) éloignée du bord de la partie principale (8), s'éloignent une pluralité de fentes (9) .

8. Pansement selon l'une des revendications précédentes, dans lequel la couche hydrophobe antimicrobienne (a) (19) comprend un tissu d'acétate de cellulose, un tissu de viscose, un tissu de coton ou un tissu mixte.

9. Pansement selon la revendication 8, dans lequel la couche (a) (19) a été traitée avec du chlorure de dialkyl carbamoyle et/ou un dimère alcène-cétène.

10. Pansement selon l'une des revendications précédentes, dans lequel la couche absorbante (b) (17) présente une teneur en eau d'au moins 20 % en poids.

11. Pansement selon l'une des revendications précédentes, dans lequel la couche absorbante (b) (17) comprend un polymère ou copolymère sélectionné parmi des monomères du groupe constitué de : acide 2-acrylamido-2-méthylpropane sulfonique-sodium (NaAMPS), acide 2-acrylamido-2-méthylpropane sulfonique-potassium (AMPS-K), acide 2-acrylamido-2-méthylpropane sulfonique-ammonium (ammonium-AMPS), potassium-3-sulfonatopropylacrylate (SPAK), sodium-B-sulfonatopropylacrylate (NaSPA), acide acrylique ou est constitué de combinaisons de ceux-ci.

12. Pansement selon l'une des revendications précédentes, dans lequel la couche de recouvrement (c) (15) comprend un film de polyuréthane.

13. Pansement selon l'une des revendications précédentes, qui comprend en outre une couche adhésive (21) sous la forme d'une bande adhésive qui s'étend le long du bord du pansement et qui est appliquée sur le côté de la couche antimicrobienne (a) (19) opposé à la couche absorbante (b) (17), dans lequel la bande adhésive ne recouvre que partiellement la couche antimicrobienne (a) (19).

14. Pansement selon l'une des revendications précédentes, qui comprend en outre une couche de protection (23) détachable.
